# EUROPEAN PATENT APPLICATION

(11) **EP 3 760 240 A1**
(43) Date of publication of application: **06.01.2021**
(21) Application number: 19756598.9
(22) Date of filing: 26.02.2019
(51) Int. Cl.: A61L 27/36, A61L 27/38, A61L 27/54, A61K 35/32

(54) **DECELLULARIZED BONE BIOMATERIAL ENRICHED WITH A HYDROGEL CONTAINING DECELLULARIZED EXTRACELLULAR BONE MATRIX**

(30) Priority: 26.02.2018 BR 102018003726
(71) Applicant: Universidade Federal do Espirito Santo - UFES, 29075-910 Viória - ES (BR)
(72) Inventor: SILVA BARROS, Rodolpho José da, Praia da Costa 29101- 094 Vila Velha - ES (BR); NOGUEIRA, Breno Valentim, Reno Jardim da Penha 29060-110 Vitória - ES (BR); SOUZA, Alex Balduino de, Icarai 27052-860 Niteroi - RJ (BR); MARANDUBA, Carlos Magno da Costa, Bairro Santa Helena 36015-350 Juiz de Fora - MG (BR); SOARES DO AMARAL, Danielle Luciana Auroro, Novo Horizonte 36038-510 Juiz de Fora - MG (BR); PINTO DE OLIVEIRA, Jairo, 29165-800 Serra - ES (BR)
(74) Representative: Potter Clarkson
(86) International application number: PCT/BR2019/000008
(87) International publication number: WO 2019/161465

(57) **Abstract**

It is a biomaterial developed from decellularized animal bone tissue and coated with a bone extracellular matrix in the form of a gel, which is capable of conferring efficient mechanical and biological support, and which can also be enriched with cells, nanocomposites or drugs, when used as a bone graft, bioreactor, or vehicle in treatments, research and development of other biomaterials; That is, it was developed from decellularized, lyophilized, porous and rigid, manipulable, safe and non-immunogenic bone material, coated and enriched with stimulating substances specific to bone tissue, presented / used in particulate or block form, thus possessing ability to promote the development of in vitro mature or progenitor cell lines when used as a bioreactor, and demonstrates a high integration capacity and a faster rate of fracture healing and filling of bone defects when used as an in vivo graft; The biomaterial also allows the promotion of cellular development, from the maintenance of the integrity of the organic extracellular matrix of the bone tissue, being able to improve healing time, reduce costs and contribute scientifically to basic research demonstrating the biotechnological importance, the investigative and applicability of decellularized organic matrices in biomaterials.

## Description

The present patent relates to a decellularized bone biomaterial enriched with bone extracellular matrix hydrogel, more specifically to a natural biomaterial, developed exclusively from decellularized, lyophilized, porous and rigid, manipulable, safe and non-immunogenic bone biomaterial, coated and enriched with stimulating substances specific for bone tissue, presented / used in particulate or block form. Thus, it has the capacity to promote the development of mature or progenitors cell lines in vitro when used as a bioreactor, and demonstrates a high integration capacity and a faster rate of fracture healing and filling of bone defects when used as a bone graft in vivo.

Until now, for bone tissue regeneration, autogenous bone grafts (autograft), followed by the use of cadaverous human bone (allograft) or animal bones (xenograft), are the most commonly used alternatives with the best results in treatments and corrections of bone failure. The search for new methods that overcome the clinical efficacy of allografts and xenografts is still a challenge for Tissue Bioengineering (SHRIVATS; MCDERMOTT; HOLLINGER, 2014).

In order to produce an ideal bone tissue framework, one of the most important criteria is that the product is composed of a highly interconnected porous network of sufficiently large sizes for cell migration, fluid exchange, and eventually tissue growth and vascularization. However, only favoring cell development is not enough, the physiological role of bone tissue requires that biomaterials implanted in defective sites are also capable of withstanding mechanical loads associated with the functional compressive stimulus of the bone, in addition to generating no immune response (GRUSKIN et al., 2012).

Deproteinized and/or lyophilized biomaterials were manufactured from natural bones and corals and have been used on a large scale. They have the advantage of inheriting the properties of the original materials as the pore structure, however, as all organic material is removed by gradual annealing (up to 300 °C) and subsequently lyophilized, a number of important substances and stimulating factors for tissue regeneration are lost. This type of inorganic porous material has gained wide acceptance for several dental and orthopedic applications, which despite being a purely mineral and osteoconductive framework, is not capable to improve bone regeneration, since it does not present a relevant osteoinductive property. In this context, demineralized bone repair materials, such as Bio-oss®, exhibit a relatively weak clinical effect by a "creeping substitution" cellular process, which limits its application to large bone defects (LEI et al., 2015). However, only in the US, more than US $ 1 billion per year is traded in the market that concentrates the use of these conventional bone graft products (GRUSKIN et al., 2012).

Currently, various biomaterials have been developed to be used as bone substitutes, and more broadly are classified as inorganic and organic materials, which include naturally derived or synthetic constituents. Inorganic materials such as beta- phosphate tricalcium (0-TCP), hydroxyapatite (HA) and bioactive glass ceramics (Bioglass®, BonAlive®) have been used for bone tissue engineering purposes because of their similarities in structure and composition with the inorganic elements of the bone itself. These inorganic biomaterials even have benefits as a potential for osteoconductivity and compression capacity, which is often equal to or greater than the bone tissue, however, because they have a naturally fragile structure, it always generates a great concern for biological applications that must withstand high loads (FERNANDEZ- YAGUE et al., 2015).

An alternative to inorganic materials are organic-natural polymers or chemically synthesized. These alternative materials have characteristics that encourage their applications in tissue engineering. Biomaterials derived from natural sources, such as collagen, hyaluronic acid, cellulose, silk, alginate and chitosan, are generally characterized by biocompatibility, allowing adhesion and migration of cells within their structures. Collagen sponges, in particular, have been used to provide growth factors and promote bone regeneration. Although of great diversity, the major limitations of natural polymers include difficulties in processing and purification, and concerns about immunogenicity. In addition, the possibility of variability of products and batches of materials decreases the predictability of results in the clinic. Finally, no naturally derived organic biomaterial is able to combine the mechanical properties of bone tissue, which contains both organic and inorganic components (SHRIVATS; MCDERMOTT; HOLLINGER, 2014).

The field of synthesis of organic polymers intended for tissue engineering has grown considerably, mainly in relation to the polymerization techniques of frameworks to minimize batch variability. Synthetic biomaterials with specific micro and macroscale characteristics are being developed. Microscale features include composition, architecture, and linkage groups, while macro-scale features include porosity, rigidity, and elasticity. As for the composition, the polymers frequently synthesized for regenerating biomaterials of bone tissue include polylactic acid (PLA), polyglycolic acid (PGA), PLGA, polycaprolactone (PCL), polyethylene (PE), polyethyleneglycol (PEG) and methyl polymethacrylate (PMMA), among others. Although biologically inspired and highly versatile, synthetic polymers also exhibit flaws as models for tissue engineering. The lack of bioactivity restricts the positive interactions between biomaterials and hosts, in the opposite way to that observed in naturally derived polymers that naturally have binding domains for tissue extracellular matrix (ECM). In addition, degradation products of synthetic polymers generally include acid by-products such as PLA or PGA that may hinder regenerative processes (SHRIVATS; MCDERMOTT; HOLLINGER, 2014).

More recently, the clinical success of some research based on the development of frameworks for bone regeneration seems to be associated with overcoming the limitations presented by monophasic biomaterials through the development of synergistic combinations of inorganic and organic biomaterials. In this sense, interesting progress has already been achieved in the search for hybrid products. The production of promising scaffolds was achieved by combining organic and inorganic materials, allowing the creation of biocompatible models, which confer some osteoinductive capacity to previously only osteoconductive materials and with the required compression strength in areas of bone defects. The combination of collagen nanofibers and polycaprolactone microfibers (PCL), for example, was achieved without compromising the adhesive properties of collagen or the mechanical resistance of PCL. The mixture of chitosan and hydroxyapatite in biomolders resulted in materials with mechanical properties, porosity and bioactivity to support cell growth and new bone formation, as seen in InFuse® products, a successful combination of biomaterial and growth factor. There are already mineral materials enriched with sources of collagen, growth factors or bone morphogenetic proteins (BMPs), especially BMP-2 that promotes osteogenic differentiation. However, despite the proven efficacy of BMP, its clinical application is still complicated because of its low biological half-life, systemic side effects and rapid removal at the site of injury. More recently, research has focused on delivery systems that minimize the diffusion of BMPs away from their therapeutic target not only to improve bone formation but also to limit unwanted reactions. Other examples of biomaterials with combined products include collagen and HA, PGA and βTCP, as well as a particularly interesting association of PEG, PCL, collagen and nano-HA (SHRIVATS; MCDERMOTT; HOLLINGER, 2014).

One of the main purposes of producing biomaterials for tissue regeneration is to support and facilitate the physiological functions needed at the site of injury. In general, this includes providing the ideal framework for the migration and specialization of the regenerative cell population, as well as the sequestration of extracellular matrix components (ECM) and local growth factors. This multidimensional support acts favoring the ability of fixation, anchorage, differentiation, proliferation and cell functionality. It is known that the extracellular matrix of mammalian tissues can be isolated, decellularized and used as scaffolds, which have already been shown to facilitate the functional restoration of different tissues. Constructive remodeling mechanisms from ECM include recruitment of progenitor cells, promotion of cell migration and proliferation, regional angiogenesis and promotion of a favorable M2 macrophages phenotype at the host tissue interface and the biological scaffolds. Although ECM has been used successfully in non-homologous sites, recent studies have demonstrated specificity, i.e., occurrence of additional functions and complex tissue formation when biological matrices were derived from specific tissues (Sawkins et al., 2013). It is also well described that in addition to differences in preparation, processing, and site of procurement, similar to other tissues, the donor's age also has a significant impact on the properties of ECM and its clinical performance (BENDERS et al., 2013; SAWKINS et al., 2013;WILLIAMS et al., 2014).

In this context, the demineralized bone matrix (DBM) was developed as a bone substitute to overcome the limitations of conventional grafts and offer greater tissue specificity at the implant site. The conductive ossicle DBM is produced by the acid extraction of the mineral content of the allogeneic or xenogenic bone and contains growth factors, non-collagenous proteins and type I collagen (SAWKINS et al., 2013). Although with variability, the osteoinductive effect of DBM has been well described in animal studies, but there is a shortage of similar information for clinical studies in humans. The final product of the demineralization process is a DBM powder generally associated with a viscous carrier, which is intended to facilitate handling, formulation and minimal clinical use as it is not effective in providing continuity and necessary physical support in the correction of defects critics. Viscous carriers are generally water-soluble polymers, such as sodium hyaluronate or carboxymethylcellulose, or anhydrous miscible solvents, such as glycerol, which may have nephrotoxic effects. Studies designed to test the use of vehicles on the effectiveness of DBM are limited. What is known so far is that there appear to be differences in osteogenic activity that may be related to the use of different vehicles, as well as the amount of DBM in suspension, and the vehicle's ability to deliver the DBM particles at the site of the bone defect for a sufficient period of time to promote bone regeneration. A recent study characterized an inflammatory response to four commercial bone graft substitutes and found that the three DBM materials produced more inflammation than a synthetic hydroxyapatite compound. However, it was not determined whether the DBM material or vehicle caused the inflammatory response (GRUSKIN et al., 2012; CHENG; SOLORIO; ALSBERG, 2014).

Just as the development of increasingly specific scaffolds seems to be central to the therapeutic success of tissue engineering, it also seems clear that the availability of multiple growth factors with temporal, spatial, and biologically stimulating dosing parameters are crucial for the development of successful regenerative therapies. Improvement of increasingly enriched biomaterials that enable drug delivery and sequential delivery of growth factors in appropriate doses may be the key to re-creating the naturally occurring bone regenerative processes during embryogenesis and healing of a fracture (SHRIVATS; MCDERMOTT; HOLLINGER, 2014).

To date, tissue bioengineering has not yet been able to produce convincing therapy for bone tissue regeneration and there are not products that integrate cell use with biomaterials (SHRIVATS; MCDERMOTT; HOLLINGER, 2014). The clinical search for new effective alternatives for bone regeneration has given rise to several possibilities that may be clinically affecting, even though none of them has prioritized the use of finely preserved decellularized bone extracellular matrices and/or enrichment with cells as therapeutic possibilities and potential by-products of tissue engineering (LI et al., 2015). The next generation of biomaterials for bone regeneration should not only physically support bone defects, but also sustain chemically and biologically the growth factors and cells present (PAUL et al., 2016).

The development of technologies such as bioreactors, which add the triad: stem cells, growth factors and scaffold, manipulated in controlled environments, have contributed to the creation of ex vivo tissues be a real possibility. The stimuli provided in 3D cultures may be able to direct cell differentiation and behavior, producing specialized tissues for implantation in vivo. Although still facing challenges regarding the standardization of techniques and product quality, there is an expectation that there will be progress in the clinical use of bioreactors, since the benefits provided by this technology, especially on the knowledge of vascularization of grafts, may be the key for the creation of more promising products and therapies (BARTNIKOWSKI et al., 2014).

Looking beyond bioreactor technology, the next generation of ex vivo tissue production could be through advances in computer-fabricated fabric technology that can combine natural and synthetic polymers as well as inorganic materials to produce biomaterials, including thermoplastics, hydrogels and more complex composite frameworks, which would allow precise control of its architecture and composition limited only by the resolution of the distribution technology. This could open doors for the production of scaffolds and tissues with geometric parameters specific to each patient, a feat inaccessible to current methods. For fabricated size, and functional fusion of the elements of the tissue engineering triad, it is imperative that basic researches are constantly advancing (FERNANDEZ-YAGUE et al., 2015).

Finally, in the insistent search for the development of biomaterials that promote bone growth, despite the demand and even if there are notable products that present some clinical success in research, to the present date, none of them was able to overcome the efficacy of the grafts autologous, homologous or xenologist in their abilities to treat critical size defects. Several biomaterials developed to have some biocompatibility, because they are designed to imitate as close as possible to the porous network of the native bone extracellular matrix, however, they still do not compare to the natural bone in terms of structure and function (SHRIVATS; MCDERMOTT; HOLLINGER, 2014).

Given above the technologies used at the time, it was possible to identify the
main limitations and drawbacks of them, such as:
- Limited availability and high morbidity to the donor for autografts;
- The low availability and quality of bone bank material;
- The chance of immunogenic rejection and potential risk of transmission of diseases by frozen/lyophilized allografts and xenografts;
- The possibility of unsatisfactory and adverse clinical results in conventional therapies;
- The relatively weak clinical effect and low osteoinductive capacity of inorganic biomaterials;
- The low capacity of support to the mechanical compression and fragility of some materials;
- The lack of standardization of preparation techniques and batch quality of the products;
- The lack of products that ensure adequate quality and availability of growth factors;
- The lack of integrated products with technologies that allow the inclusion of cells;
- The lack of compound products with multiple growth factors;
- The lack of safe products of specific origin of the bone tissue;
- The lack of safe products produced from specific regions of the bone tissue;
- The lack of safe products produced from bone tissue with specific age;
- The lack of safe products of decellularized bone matrix;
- The lack of customized products for each type of patient, injury or biological purpose;
- The low efficiency of existing bioreactor models tested;
- The lack of products intended for in vitro research;
- High cost of hybrid products, especially if associated with growth factor.

In this sense, and in order to solve or even overcome the identified drawbacks, the biomaterial of decellularized bone enriched with bone extracellular matrix hydrogel was developed. Which represents a biomaterial developed from decellularized animal bone tissue and coated with a bone extracellular matrix in the form of a gel that is capable of conferring efficient mechanical and biological support and which is further enriched with a cell line, nanocomposites or drugs, when used as a bone graft, bioreactor, or vehicle in treatments, research and development of other biomaterials. The product of this patent, unlike other technologies, has been developed since its inception to promote cell development, by maintaining the integrity of the organic extracellular matrix of the bone tissue, being able to improve the healing time, reduce costs and contribute scientifically to basic research demonstrating the biotechnological importance, the investigative need and applicability of organic matrices decellularized in biomaterials.

The decellularized bone biomaterial enriched with bone extracellular matrix hydrogel can be better understood through the detailed description in accordance with the following attached figures wherein:

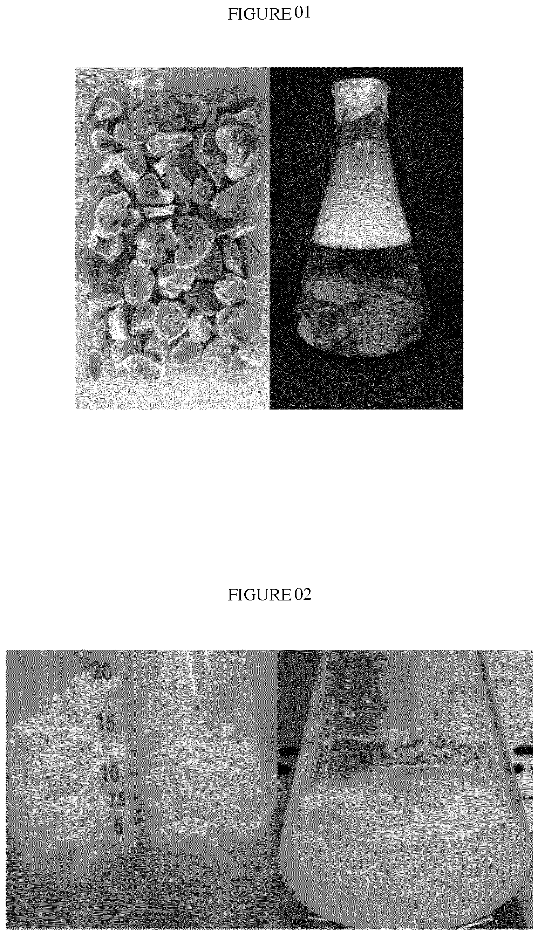

FIGURE 01 shows pictures of fragments of bone tissue before (left) and after immersion in solution (right), during the decellularization process of the decellularized bone biomaterial enriched with bone extracellular matrix hydrogel.
FIGURE 02 shows pictures of lyophilized particles (left) and hydrogel (right) of decellularized bone matrix, of decellularized bone biomaterial enriched with bone extracellular matrix hydrogel.

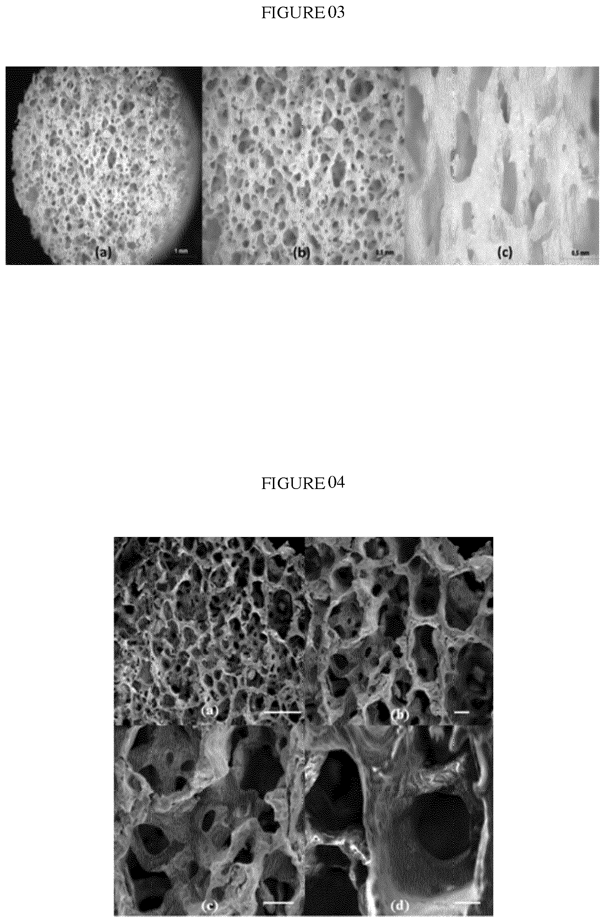

FIGURE 03 shows steromicroscopy images of the decellularized bone biomaterial with magnification of 8x (a), 12.5x (b) and 20x (c), of the decellularized bone biomaterial enriched with bone extracellular matrix hydrogel.
FIGURE 04 shows scanning microscopy images of the decellularized bone biomaterial at the magnifications of 50x (A), 100x (b), 200x (c) and 350x (d), of decellularized bone biomaterial enriched with bone extracellular matrix hydrogel.

In accordance with the above figures it can be seen that the decellularized bone biomaterial enriched with decellularized bone extracellular matrix hydrogel corresponds to a natural biomaterial, developed exclusively from decellularized, lyophilized, porous and rigid, manipulable, safe and non-immunogenic, coated and enriched with stimulating substances specific to bone tissue bones, presented/used in particulate or bulk form, with potential to be exploited by industrial production and basic science in Health Sciences (Medicine, Veterinary Medicine and Dentistry) and Biotechnology. It has the ability to promote the development of in vitro mature or progenitor cell lines when used as a bioreactor, and demonstrates high integration capacity and greater speed of fracture healing and filling of bone defects when used as an in vivo graft.

For this, the collected animal bones are of regulated origin/certified (Federal Inspection Service of the Ministry of Agriculture, Livestock and Supply - SIF / MAPA / Brazil), collected from slaughterhouse and sent to the laboratory. After cleansing and dissection of the collected bones, specific areas of the bone tissue will be selected and treated in order to preserve to the maximum the physical, biological and morphofunctional characteristics of the organic extracellular matrix and tissue present. The selected tissue will be treated by immersion in detergent solution (Triton 1-3%, Sodium Dodecyl Sulfate- SDS 0.1-2.5%, or other) under agitation of 200-500rpm for 24-96 hours until that the material presents a quantity of sample DNA of less than 50 ng. After decellularization, the resulting solid matrix will be repeatedly washed with buffer solution (PBS pH 7.4-7.8 or other), oven dried at controlled temperature between 25-50 ° C for at least 12-48 hours, then lyophilized, sterilized in ethylene oxide and preserved for gel coating.

The decellularized bone extracellular matrix gel will be produced from the same material collected. After selection of the bone tissue, the material frozen in liquid nitrogen will be ground into small fragments or until it becomes powder. Demineralization in acid solution (0.1-2.5 N HCI or other) is then performed under stirring at 200-500 rpm at room temperature for 24-96 hours and thoroughly washed with distilled water. After drying the material will be degreased in chloroform/methanol solution, under stirring at 200-500rpm, at room temperature, for 1-3 hours, and washed with distilled water insistently. After drying, the material will be decellularized by immersion in enzymatic solution (0.01% -0.5% Trypsin and 0.01-0.2% EDTA or other) under agitation of 200-500rpm at 37 ° C for 12 -48 hours, until the material has less than 50ng sample DNA. After that time, 1% antibiotic and antifungal solution (Streptomycin / Penicillin, Gentamicin, or other) will be added under agitation of 200-500rpm at 4°C for 12-48 hours.

After this period, the contents will be tested in culture against contamination, lyophilized and kept in freezer -80 °C. From the sterile lyophilized content, enzymatic digestion with acidic solution (HCI 0.01-0.1 N) of Pepsin 0.5-2.5mg/mL is performed under magnetic stirring at room temperature for 48 hours-120 hours. Thereafter, the material called digested matrix is kept in a freezer -80 °C. From the digested matrix, neutralization is performed by 0.05-0.5N NaOH solution and buffer solution (PBS pH 7.4¬7.8 or other) at 4 °C. For formation of the hydrogel the material is placed at 37 °C for at least 1-6 hours.

With the decellularized materials, solid matrix and gel produced, the biomaterial will be produced by immersing the solid matrix into the gel resulting from the decellularized matrix itself, so that the hydrogel fills the pores present therein and is capable of coating the entire material. Then the lyophilization and preservation of the biomaterial in freezer -80 °C can be carried out until the moment of use.

Given the description of the technology above, and given the preferred embodiments and possible implementations after the patent filing, follow in a way that does not tend to limit it, and there may be constructive variations that are equivalent without, however, escaping the protection scope of the invention.

## Claims

1. DESCELULARIZED BONE BIOMATERIAL ENRICHED WITH DECELULARIZED BONE EXTRACELLULAR MATRIX HYDROGEL, **characterized in that** it is obtained from natural animal or human bone, adult, young, neonate or fetal, subjected to a chemical and enzymatic decellularization process, after several stages of cleaning, sorting, grinding, immersion, washing, drying, freeze-drying and freezing;

2. DESCELULARIZED BONE BIOMATERIAL ENRICHED WITH DESCELULARIZED BONE EXTRACELLULAR MATRIX HYDROGEL according to claim 1, **characterized in that** it is obtained from decellularization processes using material collected from donors of specific ages;

3. DESCELULARIZED BONE BIOMATERIAL ENRICHED WITH DECELULARIZED BONE EXTRACELLULAR MATRIX HYDROGEL, according to claim 1 and 2, **characterized in that** it is obtained from decellularization processes using material collected from specific regions of the bone tissue such as the periosteum, the endosteum, the growth zone, the different areas of ossification, the articular surface , of the compact bone, of the spongy bone, of the medullary canal or other;

4. DESCELULARIZED BONE BIOMATERIAL ENRICHED WITH DECELULARIZED BONE EXTRACELLULAR MATRIX HYDROGEL, according to claims 1, 2 and 3, **characterized in that** it is obtained from decellularization processes using detergents such as Triton, Sodium Dodecyl Sulfate -SDS, Abietamidomethyl-diethylammonium Acetate or the like, isolated or in combination, in different concentrations, for the preservation of the extracellular matrix of tissues;

5. DESCELULARIZED BONE BIOMATERIAL ENRICHED WITH DECELULARIZED BONE EXTRACELLULAR MATRIX HYDROGEL, according to claims 1, 2, 3 and 4, **characterized in that** it is obtained from the gelled or dry decellularized extracellular matrix hydrogel coating;

6. DESCELULARIZED BONE BIOMATERIAL ENRICHED WITH DECELULARIZED BONE EXTRACELLULAR MATRIX HYDROGEL, according to claims 1, 2, 3, 4 and 5, for use as a bone graft in powder, granules of varying granulometry, block or gel form presentations;

7. DESCELULARIZED BONE BIOMATERIAL ENRICHED WITH DECELULARIZED BONE EXTRACELLULAR MATRIX HYDROGEL, according to claims 1, 2, 3, 4, 5 and 6, **characterized in that** it is for use in animal and/or human dental procedures, exodontia, rhizectomy, three-wall bone defects, bone craters, deep and narrow hemi-septics, inter-root defects and in association with other techniques for extracts, bone augmentation or implantology;

8. DESCELULARIZED BONE BIOMATERIAL ENRICHED WITH DECELULARIZED BONE EXTRACELLULAR MATRIX HYDROGEL, according to claims 1, 2, 3, 4, 5, 6 and 7, used for orthopedic procedures in animals and/or humans, for bone augmentation, correction of fractures and/or bone defects in different regions of the skeleton;

9. DESCELULARIZED BONE BIOMATERIAL ENRICHED WITH DECELULARIZED BONE EXTRACELLULAR MATRIX HYDROGEL, according to claims 1, 2, 3, 4, 5, 6, 7 and 8, **characterized in that** it is associated with nanocomposite(s), synthetic drug(s) and/or other excipient(s) suitable for use(s) for the indicated indications;

10. DESCELULARIZED BONE BIOMATERIAL ENRICHED WITH DECELULARIZED BONE EXTRACELLULAR MATRIX HYDROGEL, according to claims 1, 2, 3, 4, 5, 6, 7, 8 and 9, **characterized in that** it is for use as a bioreactor or framework for tissue engineering applications in the development/establishment of culture or lineage of mesenchymal, progenitor or mature cells;

11. DESCELULARIZED BONE BIOMATERIAL ENRICHED WITH DECELULARIZED BONE EXTRACELLULAR MATRIX HYDROGEL, according to claims 1, 2, 3, 4, 5, 6, 7, 8, 9 and 10, to be used as feedstock in 3D printing.
